# EUROPÄISCHE PATENTSCHRIFT

(11) **EP 2 203 205 B1**
(45) Veröffentlichungstag und Bekanntmachung des Hinweises auf die Patenterteilung: **06.05.2015**
(21) Anmeldenummer: 08803342.8
(22) Anmeldetag: 28.08.2008
(51) Int. Cl.: G06M 1/04, A61M 15/00, G06M 1/08

(54) **INHALIER-GERÄT**
INHALER DEVICE
INHALATEUR

(30) Priorität: 22.09.2007 DE 102007045438
(43) Veröffentlichungstag der Anmeldung: 07.07.2010
(73) Patentinhaber: BOEHRINGER INGELHEIM INTERNATIONAL GMBH, 55216 Ingelheim (DE)
(72) Erfinder: von SCHUCKMANN, Alfred, 47627 Kevelaer (DE); HOCHRAINER, Dieter, 55216 Ingelheim am Rhein (DE); HOELZL, Hubert, 55216 INGELHEIM AM RHEIN (DE)
(74) Vertreter: Simon, Elke Anna Maria
(86) Internationale Anmeldenummer: PCT/EP2008/061337
(87) Internationale Veröffentlichungsnummer: WO 2009/037085

(56) Entgegenhaltungen:
- WO-A1-03/107269
- WO-A1-2006/051073

## Beschreibung

Die Erfindung betrifft ein Handgerät zur portionierten Ausgabe sprühfähiger Substanzen, insbesondere Inhaliermedikamenten, gemäß Gattungsbegriff des Hauptanspruches.

Handgeräte der in Rede stehenden Art sind vorbekannt (WO 2006/051073). Sie finden insbesondere Anwendung in der medizinischen Aerosol-Therapie zur Behandlung von Erkrankungen der Atemwege. Die im Gehäuse gehalterte, unter Druck stehende Kartusche beinhaltet das zu inhalierende Medikament, wobei zur Freigabe bzw. zum Ausstoß desselben eine axiale Verschiebung der Kartusche in dem Gehäuse erforderlich ist. Die bekannten Lösungen sind aufwendig und bedienungsunfreundlich, insbesondere angesichts der inneren Toleranzen des Schrittschaltwerkes und der oft unruhigen Hand mancher Patienten.

Aufgabe der Erfindung ist deshalb, ein Handgerät der in Rede stehenden Art in günstiger Weise bei vereinfachtem Aufbau so auszugestalten, dass vor allem die Schaltschritte leichtgängig und sicher durchgeführt werden können.

Diese Problematik ist durch den Gegenstand des Anspruches 1 gelöst. Es ist ein Handgerät der in Rede stehenden Art geschaffen, welches bedingt durch die gewählte Konstruktion ohne größeren Platzbedarf für das Schaltwerk als auch hinsichtlich der möglichst großen Toleranzfreiheit und Zählsicherheit erhebliche Vorteile aufweist. Die Führungszapfen des Schaltfingers durchlaufen bei Betätigung nicht nur den Schrägschlitz, der verantwortlich ist für die Drehung des Schrittschaltfinger-Sternes, sondern danach noch einen axial verlaufenden Schlitzabschnitt, was neben einem Abtauchen des Skalenringes vor dem Sichtfenster eine ergänzende Federkraft der Schaltfinger aufbaut zur sicheren Rückstellung des gesamten Schrittschaltwerkes unabhängig vom Rückhub der Kartusche, welcher bekannterweise aus deren Ventilrohr-Federung resultiert. Die Schrittschaltfinger greifen in die Zahnung des von ihnen in Drehung versetzten Sonnenrades ein und in bevorzugter Weise auch der die Rückdrehung verhindernde Rastfinger. Es ist ein möglichst toleranzfreies Zusammenwirken der Bauteile gegeben.

Nachstehend ist der Gegenstand der Erfindung anhand der beigefügten Zeichnung, welche ein Ausführungsbeispiel darstellt, näher erläutert. Es zeigen:
- Fig. 1: in perspektivischer Explosionsdarstellung das Schrittschaltwerk für das erfindungsgemäße Handgerät;

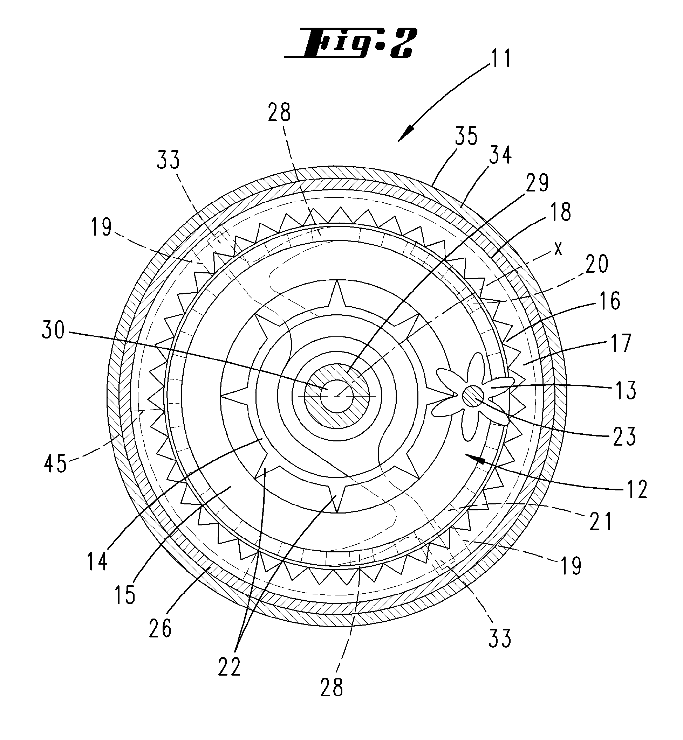

- Fig. 2: einen Querschnitt durch das Schrittschaltwerk;

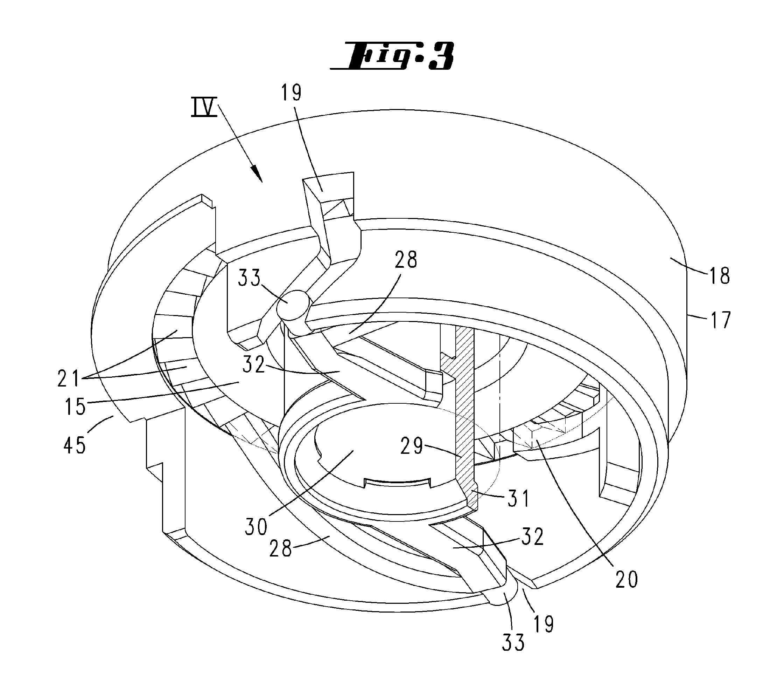

- Fig. 3: in einer schaubildlichen Darstellung den Drehantriebsbereich des Schrittschaltwerkes;

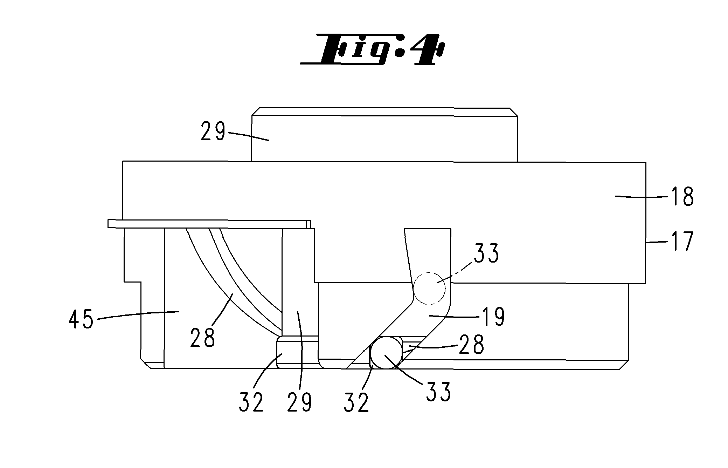

- Fig. 4: eine Seitenansicht dazu;

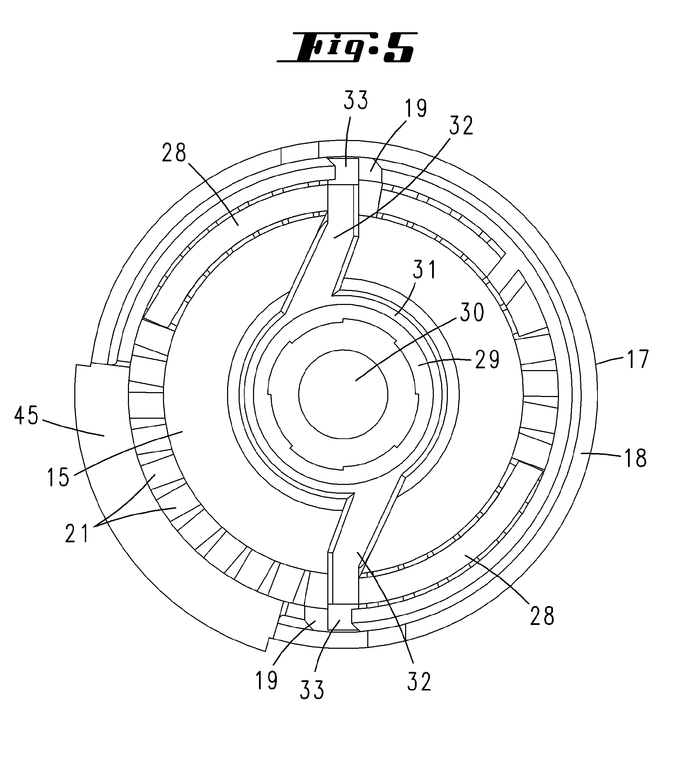

- Fig. 5: eine Unteransicht dazu;

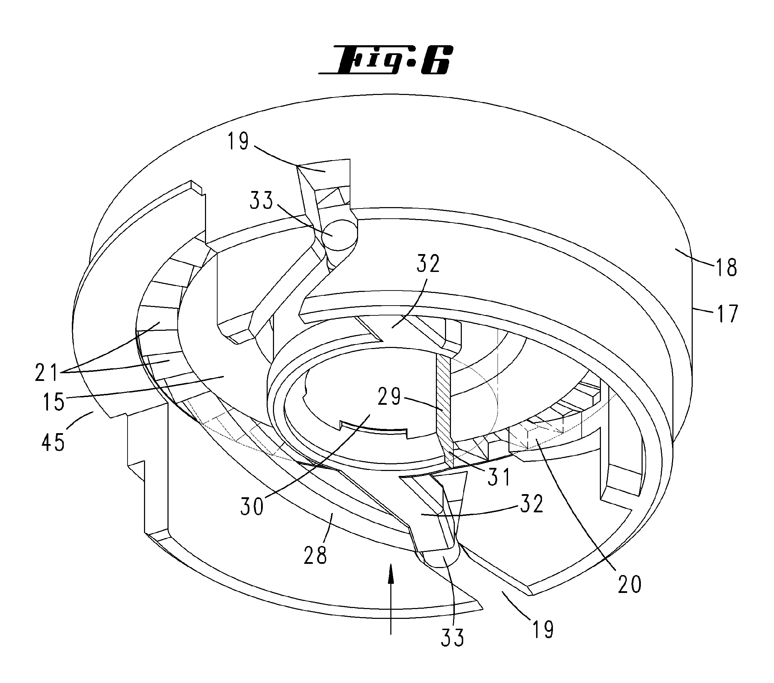

- Fig. 6: eine Darstellung wie Figur 3, jedoch teilweise verlagertem Gehäuse;
- Fig. 7: eine Darstellung wie Figur 6 bei weiterverlagertem Gehäuse;

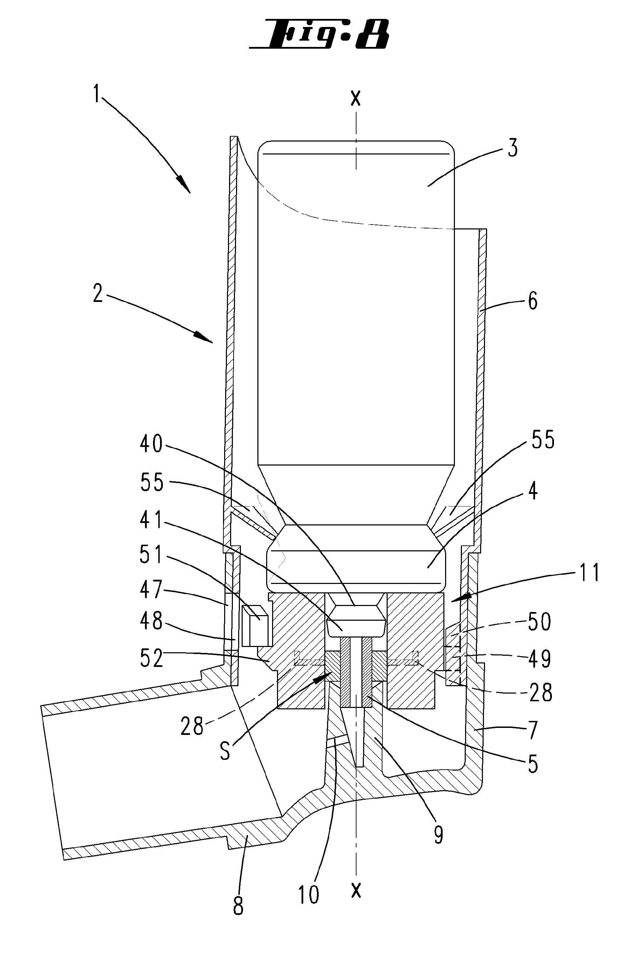

- Fig. 8: das Handgerät mit Schrittschaltwerk in der unbetätigten Stellung, schematisch;
- Fig. 9: die Darstellung der einen Entnahme-Möglichkeit.

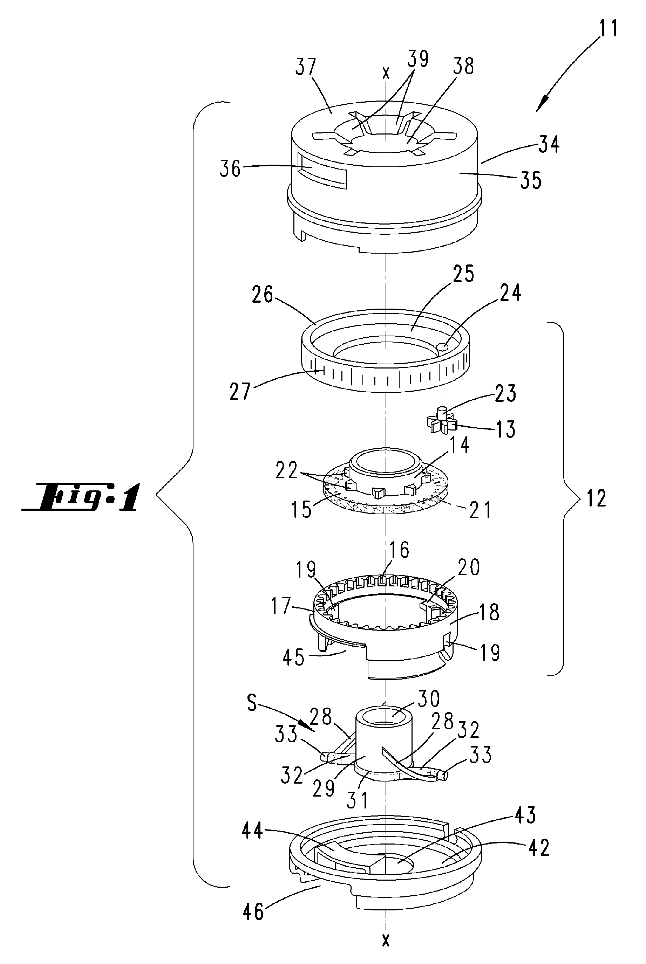

Das in Fig. 1 in einer schematischen Schnittdarstellung gezeigte Handgerät 1 dient zur portionierten Ausgabe sprühfähiger Substanzen, insbesondere von Inhaliermedikamenten.

Hierzu weist das Handgerät 1 zunächst ein Handgerätgehäuse 2 auf, in welches eine die sprühfähige Substanz beinhaltende Kartusche 3 einsetzbar ist. Diese Kartusche 3 ist in dem Gehäuse 2 axial verschiebbar.

In üblicher Weise weist der Kartuschenkopf 4 ein zentrales, sich koaxial zur Kartusche 3 erstreckendes Ventilrohr 5 auf. Über letzteres wird eine Medikamentenausgabe durch eine axiale Relativbewegung zwischen Kartusche 3 und Gehäuse 2 erreicht.

Das Gehäuse 2 ist zweigeteilt und besteht im Wesentlichen aus zwei übereinander angeordneten Ringteilen 6 und 7, von welchen das obere Ringteil 6 schaftartig ausgeformt ist und das untere Ringteil 7 ein etwa quer zur Schafterstreckung ausgerichtetes Mundstück 8 aufweist. Letzteres ist durch eine nicht dargestellte Abdeckkappe verschließbar.

Das Ventilrohr 5 der Kartusche 3 stützt sich in einem zugeordneten rohrförmigen Stützabschnitt 9 innerhalb des unteren Ringteiles 7 ab, dies bei axialer Beweglichkeit der Kartusche 3 innerhalb des die Kartusche 3 umgebenden, schaftartigen Ringteils 6.

Der das Ventilrohr 5 der Kartusche 3 klemmend aufnehmende, innerhalb des unteren Gehäuse-Ringteils 7 ausgeformte Stützabschnitt 9 ist mit einem gegenüber einen das Ventilrohrende aufnehmenden Abschnitt durchmesserverringerten Strömungskanal 10 versehen, welcher strömungstechnisch in Verbindung steht mit dem Ventilrohr 5, wobei das dem Ventilrohr 5 abgewandte Ende des Strömungskanals 10 in Richtung auf das Mundstück 8 weist.

Die beiden Ringteile 6 und 7 sind in dem dargestellten Ausführungsbeispiel steckbar miteinander verbunden. Alternativ können die beiden Teile auch über ein Gewinde, bspw. über ein Grobgewinde mit hoher Steigung, miteinander verbunden sein.

Die Anordnung der Kartusche 3 in dem Gehäuse 2 ist so gewählt, dass der Kartuschenkopf 4 in dem Gehäuse 2 etwa auf Höhe des Verbindungsbereiches zwischen Ringteil 6 und Ringteil 7 platziert ist.

Zentral unterhalb der öffnungsseitigen Stirnwand der Kartusche 3 ist in Überlappung zum Kartuschen-Ventilrohr 5 ein Schrittschaltwerk 11 angeordnet. Dieses dient zum Registrieren und Anzeigen der durchgeführten Ausgabebetätigungen, dies in Abhängigkeit von den durchgeführten Öffnungshüben der Kartusche 3.

Das Schrittschaltwerk 11 ist in Fig. 1 in einer perspektivischen Explosionsdarstellung gezeigt. Zentraler Bestandteil des Schrittschaltwerks 11 ist ein Planetenrad-Getriebe 12, bestehend aus einem Planetenrad 13, einem Sonnenrad 14, welches auf einer unterseitig gezahnten Scheibe 15 sitzt und einem mit dem Planetenrad 13 zusammenwirkenden Zahnkranz 16. Letzterer ist wandungsinnenseitig eines nicht drehbar gehalterten, rohrabschnittförmigen Ringes 17 ausgeformt. Die Mantelwandung 18 des Ringes 17 ist in diametral gegenüberliegenden Bereichen durchsetzt von schräg aufwärts in Schaltrichtung gerichtet ausgehenden Schlitzen 19, die nach unten zur dem Zahnkranz 16 abgewandten Ringkante hin offen auslaufen.

Der Zahnkranz 16 erstreckt sich in axialer Richtung etwa über die halbe Höhe des Ringes 17, dessen Mantelwandung 18 zur dem Zahnkranz 16 abgewandten Ringstirnkante hin abgestuft, sich radial verjüngend ausgebildet ist.

Unterhalb der Scheibe 15 ist innenseitig der Mantelwandung 18 des Ringes 17 ein Rastfinger 20 angeformt. Dieser ist mit Bezug auf einen Grundriss des Ringes gegenüber dem Zahnkranz 16 nach radial innen versetzt; greift entsprechend in einen zum Zahnkranz 16 nach radial innen eingezogenen Kreisraum. Weiter ist die Anordnung des in etwa in Vertikalrichtung elastisch ausgebildeten Rastfingers 20 so gewählt, dass dieser von unten in die Zähne 21 eingreift.

Der Durchmesser der das Sonnenrad 14 tragenden Scheibe 15 ist geringfügig kleiner gewählt, als der Innendurchmesser des Ringes 17 im Bereich des Zahnkranzes 16. Sonnenrad 14 und Scheibe 15 sind bevorzugt einstückig, materialeinheitlich ausgebildet.

Die unterseitig der Scheibe 15 vorgesehenen Zähne 21 sind randbezogen umlaufend und als Sägezahnung 21 vorgesehen.

Das Sonnenrad 14 weist eine grobe Verzahnung auf. So sind in dem dargestellten Ausführungsbeispiel über den Umfang des Sonnenrades 14 acht Sonnenrad-Zähne 22 gleichmäßig verteilt ausgeformt. Diese Zähne 22 wirken im Zuge der Sonnenrad-Umdrehung mit dem in derselben Ebene zwischen dem Sonnenrad 14 und dem Zahnkranz 16 des Ringes 17 angeordneten Planetenrad 13 zusammen.

Das Planetenrad 13 besitzt einen einseitig nach oben, d. h. von der Scheibe 15 des Sonnenrades 14 weg abragenden Achszapfen 23. Dieser ist in einer Bohrung 24 im Bereich eines scheibenartig nach radial innen weisenden Kragens 25 eines Skalenringes 26 drehbar gefasst. Der Skalenring 26 ist mantelaußenwandig mit einer umlaufenden Skaleneinteilung 27 versehen, wobei die Skaleneinteilung jeweils mehreren Einzel-Drehschritten des den Skalenring 26 weiterführenden Planetenrades 13 entspricht.

Die schrittweise Verlagerung des Sonnenrades 14 bzw. der einstückig hiermit ausgeformten Scheibe 15 erfolgt über etwa vertikal federnd ausweichbar ausgebildete Schrittschaltfinger 28. Diese greifen unterseitig durch in die Sägezahnung 21 der Scheibe 15 ein.

Die Schrittschaltfinger 28 sind mit Bezug zu der Hauptachse x des gesamten Schrittschaltwerkes 11 diametral gegenüberliegend angeordnet. Hierzu ist zunächst ein zylinderförmiger Zentralkörper in Form einer Nabe 29 vorgesehen mit einer zentralen axialen Durchgangsbohrung 30. Deren Durchmesser ist geringfügig größer bemessen als der Außendurchmesser des diese Durchgangsbohrung 30 zu durchsetzenden Kartuschen-Ventilrohres 5.

Fußseitig geht die Nabe 29 über in einen radial erweiterten Kragen 31. An diesem sind diametral gegenüberliegend in Radialrichtung abragende Führungsabschnitte 32 angeformt, die jeweils im Bereich ihrer freien Enden einen in dem zugeordneten Schlitz 19 des Ringes 17 einliegenden Führungszapfen 33 ausformen.

Die Schrittschaltfinger 28 wurzeln jeweils mit einem Horizontalabschnitt an den Führungsabschnitten 32 unter Belassen der über den horizontalen Abschnitt radial außen abragenden Führungszapfen 33. Die von den Horizontalabschnitten abragenden Schrittschaltfinger 28 gehen schräg aufwärts gerichtet aus, etwa unter Einschließen eines Winkels von 45 Grad zur Horizontalen, angepasst an die Schrägung der Schlitze 19 im Ring 17. Der so gebildete Schrittschaltfinger-Stern trägt das Bezugszeichen S.

Der Schrittschaltfinger-Stern S, der den inneren Zahnkranz 16 aufweisende Ring 17, die einstückig mit dem Sonnenrad 14 ausgeformte Scheibe 15 sowie der Skalenring 26 sind konzentrisch zueinander auf der Achse x ausgerichtet, wobei die Höhe des Ringes 17 so gewählt ist, dass sowohl der Schrittschaltfinger-Stern S als auch das Sonnenrad 14 samt Scheibe 15 in diesem aufgenommen sind.

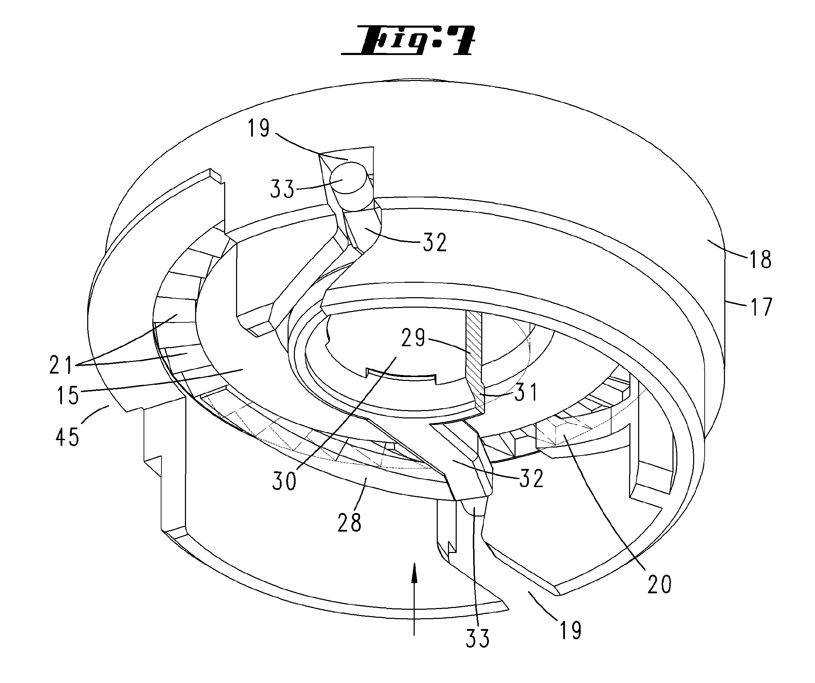

Die Schlitze 19 setzen sich aus zwei Abschnitten zusammen, von der einer schräg zur Längsachse x-x gerichtet ist und die Drehung der Schrittschaltfinger erzwingt und der zweite, sich daran anschließende Abschnitt, parallel zur Längsachse x-x gerichtet ist. Letzterer weitet sich zum oberen Ende etwas auf, so dass die Führungszapfen 33 dort mit Spiel eintreten (siehe Figur 7), insbesondere entgegen der Drehrichtung, welche der erste schrägverlaufende Abschnitt des Schlitzes 19 erzwingt. Der Hub, welchen der Schrittschaltfinger-Stern in dem geradlinig aufwärts gerichteten Schlitzabschnitt erlaubt, bringt eine weitere Spannung des inneren Federrings der Schrittschaltfinger 28 - ohne jede Drehung desselben - wodurch die Kraft - nach Loslassen der Kartusche - für das Rückstellen des Schrittschaltwerks in die Ausgangslage erheblich erhöht wird.

Das gesamte Planeten-Getriebe 12, sowie der Schrittschaltfinger-Stern S und der Skalenring 26 sind aufgenommen in einem topfartigen Schrittschaltwerkgehäuse 34 mit einem Außendurchmesser, welcher an den Außendurchmesser der Kartusche 3 angepasst ist.

Das Gehäuse 34 besitzt eine Mantelwandung 35. Diese weist ein Sichtfenster 36 auf, durch welches die Skaleneinteilung 27 des Skalenrings 26 erkennbar ist.

Die Gehäusedecke 37 besitzt eine zentrale Durchbrechung 38, welche in der in Figur 1 gezeigten Ausführungsform umfasst ist von konisch zum Gehäuseinnern hin zulaufenden Rast-Federzungen in der in Figur 1 gezeigten Ausführungsform 39. Der Durchbrechungs-Durchmesser ist angepasst an einen Durchmesser eines Taillenabschnittes 40 eines über die öffnungsseitige Stirnwand der Kartusche 3 zentral überragenden Kragens 41, aus welch letzterem das Ventilrohr 5 auswächst.

Der Gehäuseboden 42 ist gebildet durch ein gesondertes Teil. Dieses ist unter Aufnahme der vorbeschriebenen Schrittschaltwerk-Einzelteile mit dem Gehäuse 34 verbunden, so bspw. mit diesem verschweißt oder über eine Presspassung an diesem gehaltert.

Der tellerartige Gehäuseboden 42 besitzt eine zentrale Bohrung zum Durchtritt des Ventilrohres 5. Des Weiteren ist auf dem Gehäuseboden 42 ein Raststück 44 ausgeformt, welches zur lageorientierten Fesselung des Ringes 17 in eine entsprechend in dessen Mantelwandung 18 ausgeformte, fensterartige Ausnehmung 45 greift.

Im selben Winkelbereich, in welchem das Passstück 44 auf dem Boden angeordnet ist, weist die Mantelaußenwandung des Gehäusebodens 42 einen Freischnitt 46 auf. Diese ist im Einbauzustand dem Bereich des Ausgangsquerschnittes des Strömungskanals 10 im unteren Ringteilgehäuse 7 zugeordnet.

Die Funktionsweise des Schrittschaltwerkes 11 ist wie folgt:
Die Schaltglieder (Schrittschaltfinger-Stern S, Ring 17, Scheibe 15, Planetenrad 13 und Skalenring 26) wie auch das Gehäuse 34 mit dem Gehäuseboden 42 sind auf Achsen angeordnet, die sich in Längsrichtung x-x der Kartusche 3 erstrecken. So sind mit Ausnahme des Planetenrades 13 alle weiteren Bauteile des Schrittschaltwerkes 11 auf der Kartuschenlängsachse x - x positioniert.

Das Schrittschaltwerk 11 ist entsprechend konzentrisch zum Ventilrohr 5 im Schatten der Kartusche 3 innerhalb des Gehäuses 2 angeordnet, dies konkret in dem zwischen Kartuschenkopf 4 und Stützabschnitt 9 des Gehäuses 2 belassenen Bauraum. Das Schrittschaltwerk 11 stützt sich mit der in dem Schaltwerkgehäuse 34 zentral gelagerten Nabe 29 des Schrittschaltfinger-Sterns S auf der Stirnfläche des Stützabschnitts 9 des Inhaliergehäuses 2 ab. Die zentrale Achse x des Schnittschaltwerks 11 wird von der Körperachse der Kartusche 3 übernommen. Das die Nabe 29 durchsetzende Ventilrohr 5 bietet eine zusätzliche Zentrierung der gesamten Schrittschaltwerk-Einheit.

Bei Durchführung eines Betätigungshubs der Kartusche 3 und damit einhergehender Vertikalverlagerung derselben in Richtung auf den Stützabschnitt 9 wird das Schaltwerkgehäuse 34 über den Kartuschenkopf 4 vorgeschoben, dies unter Relativverlagerung des Gehäuses 34, des Planetenrad-Getriebes 12 und des Skalenringes 26 zu dem Schrittschaltfinger-Stern S, welcher eine Abstützung auf dem Stützabschnitt 9 erfährt. Infolgedessen bewirken die sich spannenden Schrittschaltfinger 28 durch Drehaufgleiten des Schrittschaltfinger-Sterns S in den mantelwandseitigen Schrägabschnitten der Schlitze 19 des Ringes 17 einen schrittweisen Drehvorschub der sägeverzahnten Scheibe 15. Einhergehend damit dreht sich das Sonnenrad 14 um denselben Winkelbetrag. Die Schrittschaltfinger 28 bewegen sich hierbei aus der schrägen Ausrichtung in Richtung auf eine senkrecht zur Längsachse x - x ausgerichtete Ebene.

Dadurch bedingt, dass das Sonnenrad 14 lediglich acht gleichmäßig über den Umfang verteilt angeordnete Zähne aufweist, führt nicht jede Schritt-Drehbewegung des Sonnenrades 14 zwangsläufig zu einer Drehbewegung des Planetenrades 13. Vielmehr wird die Drehung des Planetenrades 13 um dessen Achse und eine die damit einhergehende Drehverlagerung des Skalenringes 26 erst nach mehreren Einzel-Drehschritten des Sonnenrades 14 durchgeführt. Anschließend braucht die weitere Verlagerung der Kartusche 3, dass die Führungszapfen 33 sich in den in Längsrichtung x-x verlaufenden Abschnitten des Schlitzes 19 verlagern, was - ohne ergänzende Drehung des Schrittschaltfinger-Sternes - eine weitere Vergrößerung der in den Fingern 28 gespeicherten Rückstellkraft bewirkt.

Zugeordnet zu dem gehäuseseitigen Sichtfenster 36 weisen die zugeordneten Abschnitte der Gehäuseringteile 6 und 7 gleichfalls Sichtfenster 47, 48 auf, welche durch die gewählte Position, zugewandt dem Mundstück 8 des Gehäuses 2, im Blickfeld des das Handgerät 1 bedienenden Benutzers liegen. Zur lageorientierten Einführung des Schrittschaltwerkes 11 ist dieses mit einem radial von dem Gehäuse 34 abragenden Führungsblatt 49 versehen, welches in eine nicht näher dargestellte Vertikalnut im Inneren des Gehäuses 2, den Verschiebeweg bei Hubbetätigung zulassend eingreift.

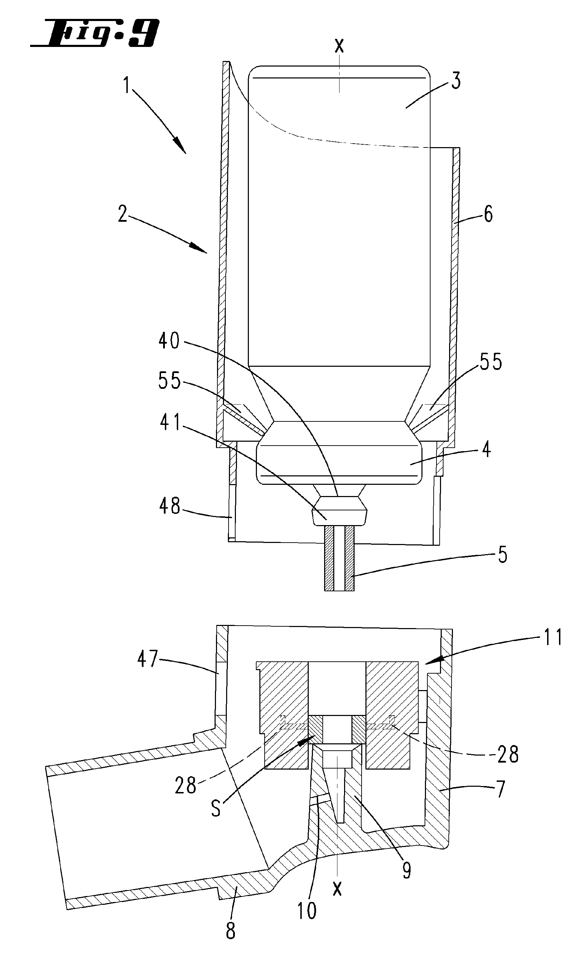

Es kann auch eine Verrastung zwischen Schrittschaltwerk 11 und Kartusche 3 im Bereich des kartuschenkopfseitigen Kragens 41 so gewählt sein, dass bei einer Entnahme der Kartusche 3 aus dem Gehäuse 2 das Schrittschaltwerk 11 an der Kartusche 3 verbleibend mit ausgezogen wird. Es kann aber ebenso, wie z.B. in Figur 9 dargestellt, die Kartusche und das Schrittwerk getrennt entnehmbar sein, insbesondere weil das Schrittschaltwerk hinsichtlich Rückfederung autark ist.

Alle offenbarten Merkmale sind (für sich) erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen.

## Patentansprüche

1. Handgerät (1) zur portionierten Ausgabe sprühfähiger Substanzen, insbesondere Inhaliermedikamenten, mit einer durch Drücken an einem Gehäuse in die Ausgabe-Stellung verschiebbaren Kartusche (3) und einem beim Öffnungshub der Kartusche (3) von dieser mitbewegten Schrittschaltwerk (11) zum Registrieren und Anzeigen durchgeführter Ausgabebetätigungen, welches Schrittschaltwerk (11) einen konzentrisch zum Ventilrohr (5) vor einem Sichtfenster (36, 47, 48) umlaufenden Skalenring (26) und eine unterseitig gezahnte Scheibe (15) aufweist, in die bei axialer Verlagerung des Schrittschaltwerkgehäuses (34) Schrittschaltfinger (28) angreifen, welche von in Schrägschlitzen (19) beweglichen Führungszapfen (33) eine Drehbewegung aufgezwungen ist, **dadurch gekennzeichnet, dass** der Schrägverlauf der Schlitze (19) sich nur über einen Teil der Hubhöhe des Schrittschaltwerk-Gehäuses (34) erstreckt und sich die Führungszapfen (33) über den Resthub des Schrittschaltwerkes (11) parallel zur Schrittschaltwerk-Längsachse (x-x) verlagern.

2. Handgerät nach Anspruch 1, **dadurch gekennzeichnet, dass** die Führungszapfen über den Resthub in den Schlitzen mit Spiel geführt sind.

3. Handgerät nach Anspruch 1, **gekennzeichnet durch** einen Rastfinger (20) der im Bereich zwischen den beiden Schrittschaltfingern (28) in dieselbe Zahnung (21) eingreift wie die Schrittschaltfinger (28).

4. Handgerät nach Anspruch 1, **dadurch gekennzeichnet, dass** den Schrittschaltfingern eine dem Öffnungshub entgegengesetzte Federverspannung innewohnt.

5. Handgerät nach Anspruch 1, **dadurch gekennzeichnet, dass** das Schrittschaltwerkgehäuse im Inhaliergehäuse linear geführt ist.

6. Handgerät nach Anspruch 5, **dadurch gekennzeichnet, dass** die Führung durch ein Führungsblatt (49) in einer Vertikalnut (52) erzielt ist.

## Claims

1. Hand-held device (1) for metered dispensing of sprayable substances, particularly medicaments for inhalation, having a cartridge (3) that is movable into the dispensing position by pressing on a housing and a step-by-step indexing mechanism (11) that is also moved by the cartridge (3) during the opening stroke of said cartridge (3), for registering and displaying the number of dispensing actuations carried out, said step-by-step indexing mechanism (11) having a graduated collar (26) arranged circumferentially, concentrically with respect to the valve tube (5), in front of an inspection window (36, 47, 48), and a disc (15) with teeth on its underside into which step-by-step indexing pawls (28) engage as the housing (34) of the step-by-step indexing mechanism is axially moved, said step-by-step indexing pawls (28) are forced into a rotary movement by guide pins (33) movable in diagonal slots (19), **characterised in that** the diagonal path of the slots (19) extends over only part of the stroke height of the step-by-step indexing mechanism housing (34) and the guide pins (33) move over the remainder of the stroke of the step-by-step indexing mechanism (11) parallel to the longitudinal axis (x-x) of the step-by-step indexing mechanism.

2. Hand-held device according to claim 1, **characterised in that** the guide pins are guided in the slots with play over the remainder of the stroke.

3. Hand-held device according to claim 1, **characterised by** a latching pawl (20) which engages in the region between the two step-by-step indexing pawls (28) in the same teeth (21) as the step-by-step indexing pawls (28).

4. Hand-held device according to claim 1, **characterised in that** a spring tension that opposes the opening stroke is inherent in the step-by-step indexing pawls.

5. Hand-held device according to claim 1, **characterised in that** the step-by-step indexing mechanism housing is guided in linear manner in the inhaler housing.

6. Hand-held device according to claim 5, **characterised in that** the guiding is achieved by means of a guide fin (49) in a vertical groove (52).

## Revendications

1. Appareil à main (1) pour la distribution par portion de substances pulvérisables, en particulier des médicaments à inhaler, comportant une cartouche (3) pouvant être déplacée par pression sur un boîtier dans la position de distribution, et un mécanisme pas à pas (11) déplacé en même temps lors de la course d'ouverture de la cartouche (3) par celle-ci pour enregistrer et afficher des actionnements de distribution réalisés, lequel mécanisme pas à pas (11) présente une bague graduée (26) tournant de façon concentrique par rapport au tube à soupape (5) devant une fenêtre de visualisation (36, 47, 48) et un disque denté (15) côté inférieur, dans lequel, lors d'un déplacement axial du boîtier de mécanisme pas à pas (34), des doigts de fonction pas à pas (28) viennent en prise, par lesquels, à partir de tenons de guidage (33) mobiles dans des fentes obliques (19), un mouvement de rotation est imposé, **caractérisé en ce que** le parcours oblique des fentes (19) ne s'étend que sur une partie de la hauteur de course du boîtier du mécanisme pas à pas (34) et les tenons de guidage (33) se décalent par la course résiduelle du mécanisme pas à pas (11) parallèlement à l'axe longitudinal du mécanisme pas à pas (x-x).

2. Appareil à main selon la revendication 1, **caractérisé en ce que** les tenons de guidage sont conduits par la course résiduelle dans les fentes avec du jeu.

3. Appareil à main selon la revendication 1, **caractérisé par** un doigt d'arrêt (20) qui est en prise dans la zone entre les deux doigts de fonction pas à pas (28) dans la même denture (21) que les doigts de fonction pas à pas (28).

4. Appareil à main selon la revendication 1, **caractérisé en ce qu'**une tension de ressort opposée à la course d'ouverture est intrinsèque aux doigts de fonction pas à pas.

5. Appareil à main selon la revendication 1, **caractérisé en ce que** le boîtier de mécanisme pas à pas est conduit de façon linéaire dans le boîtier d'inhalation.

6. Appareil à main selon la revendication 5, **caractérisé en ce que** le guidage est obtenu par une feuille de guidage (49) dans une rainure verticale (52).
